# EUROPEAN PATENT SPECIFICATION

(11) **EP 4 559 415 B1**
(45) Date of publication and mention of the grant of the patent: **19.08.2026**
(21) Application number: 24198149.7
(22) Date of filing: 03.09.2024
(51) Int. Cl.: A61B 17/3211, A61B 17/3213, A61B 90/30

(54) **TRANSPARENT SCALPEL HANDLE AND BAYONET WITH UNIVERSAL BLADE HOLDER WITH OPTICAL LENSES**
TRANSPARENTER SKALPELLGRIFF UND BAJONETT MIT UNIVERSELLER KLINGENHALTERUNG MIT OPTISCHEN LINSEN
MANCHE DE SCALPEL TRANSPARENT ET BAÏONNETTE AVEC SUPPORT DE LAME UNIVERSEL AVEC LENTILLES OPTIQUES

(30) Priority: 22.11.2023 US 202318517651
(43) Date of publication of application: 28.05.2025
(73) Proprietor: Nano Surgical Inc., Delray Beach, FL 33444 (US)
(72) Inventor: Hacker, Steven M., Delray Beach, 33444 (US)
(74) Representative: Fanfani, Stefano

(56) References cited:
- WO-A1-2019/161317
- CN-U- 202 044 318
- US-A1- 2011 060 332
- US-A1- 2012 316 553
- US-S- D 938 032

## Description

### Field of the invention

The invention relates to a transparent scalpel handle and bayonet with universal blade holder. The scalpel handle and bayonet with universal blade holder can be light transmissible and disposable. The transparent scalpel handle and bayonet with universal blade holder may comprise one or more integrated optical lenses that enable multi-directional and broad dispersion of light to provide surface area for amplification of light to a surgical site and also the surrounding area. The integrated lenses may be convex or they may have a flat surface. An illumination source, or multiplicity of illumination sources, can be located within the body of the scalpel handle and/or the bayonet with universal blade holder so that light is transmitted through the scalpel handle and the bayonet with universal blade holder. A light pipe may be integrated in the bayonet with universal blade holder.

### Background of the invention

Physicians and surgeons use a scalpel blade attached to a scalpel handle to cut through human tissue for a variety of purposes. The scalpel handle is typically one-piece and may have various shapes. At the end furthest from the scalpel handle grip typically there is an opaque end with an opaque stainless-steel bayonet with universal blade holder for attachment of the scalpel blade. The body of the scalpel handle is typically opaque stainless steel or other material.

Many shapes and materials are known to be used for scalpel handles. Scalpel handles may be round or flat and may be manufactured from a wide variety of materials including plastic, stainless steel and aluminum. Some scalpel handles include an illumination source.

Light pipes, or light guides, are tubes or optical fibers that transmit light from a light source, such as an LED to focus, diffuse or redirect that light at a desired interface. Light pipes can transmit light around corners and tight spaces with minimal loss of light intensity. In operation, one end of a light pipe is placed very closely to an LED light source on one end of a PCB board to capture emitted light and carry the rays of light emitted by the LED light source to the desired destination. The light pipe may be lined in whole or in part with a reflective coating to distribute light from the LED source to a particular area, sometimes called a light guide.

U.S. Patent No. 9072541 titled SURGICAL SCALPEL HANDLE WITH ILLUMINATOR discloses a surgical scalpel handle with illuminator allowing the surgeon to use the illumination to light the surgical field while holding the scalpel handle. Utility model CN202044318U discloses a scalpel with transparent acrylic rods arranged on both sides of the handle wherein a light source is installed at the rear end of each transparent acrylic rod.

U.S. Patent No. 10610257 titled STRUCTURAL ELEMENT WITH BAYONET HAVING A UNIVERSAL BLADE FITTING WITH ELECTRICAL SOURCE POD AND TERMINAL CONFIGURATION discloses an assembly having a support element with a bayonet having a universal blade fitting inserted into the interior of a tube and an electrical source pod.

U.S. Design Patent No. D928956 titled SCALPEL discloses an ornamental design for a scalpel with an attached blade.

U.S. Design Patent No. D938032 titled SCALPEL HAVING A TRANSPARENT UNIVERSAL BLADE FITTING discloses an ornamental design for a support element for a scalpel having a transparent universal blade fitting.

### Brief summary of the invention

The invention provides a scalpel handle and bayonet with universal blade holder according to claim 1. Further embodiments of the invention are provided in the dependent claims.

The invention relates to a scalpel handle and bayonet with universal blade holder having one or more integrated lenses and one or more illumination sources. More particularly, the scalpel handle and/or the bayonet with universal blade holder comprise at least in part a transparent material through which light from one or more illumination sources disposed in the interior of the scalpel handle and/or bayonet with universal blade holder can pass. The scalpel handle and/or bayonet with universal blade holder may each comprise one or more integrated lenses to direct the path of the light from the one or more illumination sources contained within the handle body and the bayonet portion of the handle as desired by the user.

The one or more illumination sources may be disposed in the interior of the scalpel handle; at the end of the scalpel handle proximate the bayonet with universal blade fitting; within the bayonet with universal blade holder; within the tip of the bayonet with universal blade holder; or combinations thereof. At least one power source and an on/off device are disposed in the scalpel handle or the bayonet with universal blade holder that are in communication with the one or more illumination sources.

In one embodiment, one or more light pipes are disposed along the longitudinal axis of the bayonet with universal blade holder extending from one or more of the illumination sources. In one embodiment, the one or more light pipes transmit light from the one or more illumination sources past a scalpel blade attached to the bayonet with universal blade fitting and may be designed to distribute light along the area of the scalpel blade to reduce shadowing that may occur due to the opaque nature of the scalpel blade.

In one embodiment, a light transmissible scalpel handle and bayonet with universal bayonet holder comprises a circuit including one or more batteries, wires, PCB mounted resistors, one or more PCBs and one or more light sources mounted to the one or more PCBs disposed in the interior of the scalpel handle. In one embodiment, one or more illumination sources may be used to either pass light through the scalpel handle or bayonet holder with universal blade holder in a multitude of directions. Additional light sources may be used as indicator lights as to status of scalpel handle operations, such as on and off positions.

### Brief description of the drawings

The various features of the invention and the manner of attaining them will be described in greater detail with reference to the following description, claims, drawings, wherein like designations denote like elements.
FIG. 1 is a perspective view of a scalpel handle and bayonet with universal blade holder with a light pipe according to one embodiment of the invention.
Fig 2 is a perspective view of scalpel handle and bayonet with universal blade holder with electric circuit and illumination source according to one embodiment of the invention.
FIG. 3 depicts a front view of integrated lenses comprising convex lenses according to the embodiment shown in FIG. 2.
FIG. 4 is a perspective view of a scalpel handle and bayonet with universal blade holder with a light pipe according to one embodiment of the invention.
Fig 5 is a perspective view of scalpel handle and bayonet with universal blade holder with a flex PCB and illumination source according to one embodiment of the invention.
FIG. 6 depicts a front view of integrated lenses comprising convex lenses according to the embodiment shown in FIG. 5.
FIGS. 7 and 8 depict a front view of alternative profiles of integrated lenses suitable for use comprising concave and flat integrated lenses according to an example not part of the present invention.
FIG. 9 is a perspective view of a scalpel handle and bayonet with universal blade holder having a light pipe.
Fig 10 is a perspective view of scalpel handle and bayonet with universal blade holder having an electrical circuit and illumination source according to one embodiment of the invention.

### Detailed description of the invention

The invention relates to a transparent scalpel handle and bayonet with universal blade holder. The transparent scalpel handle and bayonet with universal blade holder at least in part comprises a transparent material through which light from one or more illumination sources can pass. In one embodiment, the transparent scalpel handle and bayonet with universal blade holder may comprise one or more integrated lenses integrated into the scalpel handle and/or the bayonet and/or the universal blade holder that enable multi-directional and broad dispersion of light to provide surface area for amplification of light to a surgical site and also the surrounding area.

The term "integrated lenses" means a lens that is an integral part of the outer or inner surface of the scalpel handle and/or bayonet and/or the universal blade holder, such that light that is emitted by an illumination source disposed within the scalpel handle and/or bayonet with universal blade holder is directed to focus or diverge according to the shape of the lens. The shape of the lens may be defined from the viewpoint of the interior of the scalpel handle and/or bayonet. For example, light passing through a concave lens will cause light emitted from an illumination source in the interior of the scalpel handle and/or bayonet to diverge in the environment after passing through the lens. Light passing through a convex lens will cause light emitted from an illumination source in the interior of the scalpel handle and/or bayonet to converge in the environment after passing through the lens. Light passing through a flat lens will cause light emitted from an illumination source in the interior of the scalpel handle and/or bayonet to pass essentially in a straight line into the environment after passing through the lens. The extent that light diverges or converges is determined by the amount of concavity or convexity and size of the lens and can be determined as desired by a user. One or more of the integrated lenses may comprise a transparent optical device with curved surfaces (concave or convex) to focus or diverge light. One or more of the integrated lenses may comprise a substantially flat surface to direct light straight towards a target. The one or more integrated lenses enable multi-directional and broad dispersion of light giving a surgeon's need for an enlarged illuminated surface area for amplification of light to not only a surgical site but also surrounding area.

In one embodiment, the scalpel handle comprises one or more integrated lenses. In one embodiment, the bayonet with universal blade holder comprises one or more integrated lenses. In one embodiment, the scalpel handle and the bayonet with universal blade holder each comprise one or more integrated lenses.

In one embodiment, one or more of the integrated lenses are integrated into the inner surfaces of the scalpel handle and bayonet with universal blade holder. In one embodiment, one or more of the integrated lenses are fixed and integrated on the inner surfaces of the scalpel handle and the bayonet with universal blade holder. In one embodiment, one or more of the integrated lenses are detachable from the inner surfaces of the scalpel handle and the bayonet with universal blade holder.

In one embodiment, one or more of the integrated lenses are integrated into the outer surfaces of the scalpel handle and bayonet with universal blade holder. In one embodiment, one or more of the integrated lenses are fixed and integrated on the outer surfaces of the scalpel handle and the bayonet with universal blade holder. In one embodiment, one or more of the integrated lenses are detachable from the outer surfaces of the scalpel handle and the bayonet with universal blade holder.

In one embodiment, one or more of the integrated lenses are integrated into the wall of the scalpel handle and bayonet with universal blade holder. In one embodiment, one or more of the integrated lenses are fixed and integrated in the wall of the scalpel handle and the bayonet with universal blade holder.

In one embodiment, one or more of the integrated lenses are disposed to transmit light from one or more illumination sources circumferentially from the scalpel handle and/or bayonet with universal blade holder. In one embodiment, one or more of the integrated lenses are disposed to amplify light from the one or more illumination sources circumferentially from the scalpel handle and/or bayonet with universal blade holder. In one embodiment, one or more of the integrated lenses are disposed to diverge light from the one or more illumination sources circumferentially from the scalpel handle and/or bayonet with universal blade holder. In one embodiment, one or more of the integrated lenses are disposed to converge light from the one or more illumination sources circumferentially from the scalpel handle and/or bayonet with universal blade holder. In one embodiment, a substantially flat integrated lens is disposed on the tip of the bayonet portion of the handle or proximate to the bayonet portion of the handle and distal from the scalpel handle and is disposed to amplify light in a straightforward pattern toward the bayonet with universal blade holder from one or more illumination sources.

In one embodiment, one or more illumination sources are disposed in the interior of the scalpel handle. In one embodiment, one or more illumination sources are disposed at the end of the scalpel handle proximate the bayonet with universal blade holder. In one embodiment, one or more illumination sources are disposed in the interior of the scalpel handle; at the end of the scalpel handle proximate; within the bayonet with universal blade holder; within the tip of the bayonet with universal blade holder; or combinations thereof. In each embodiment, at least one power source and an on/off device are disposed in the scalpel handle or the bayonet with universal blade holder that are in communication with the one or more illumination sources. In one embodiment, one or more of the illumination sources comprises an LED bulb. In one embodiment, one or more of the illumination sources comprises an LED bulb that transmits blue light. In one embodiment, the LED bulb transmits therapeutic blue light at a wavelength between about 380 nm and 500 nm.

In one embodiment, one or more light pipes are disposed along the longitudinal axis of the bayonet with universal blade holder extending from one or more of the illumination sources. In one embodiment, one or more light pipes are in communication with one or more of the illumination sources and transmit light emitted by the one or more illumination sources. In one embodiment, the one or more light pipes transmit light from the one or more illumination sources past a scalpel blade attached to the bayonet with universal blade fitting. In one embodiment, one or more light pipes transmit light from one or more illumination sources to emit light in the area of a scalpel blade attached to the bayonet with universal blade fitting. In one embodiment, one or more light pipes are in direct contact with one or more of the illumination sources. In one embodiment, one or more light pipes are in indirect contact with one or more of the illumination sources.

In one embodiment of the invention, the scalpel handle contains one or multiple illumination sources and has an integrated convex lens to amplify light around the surgical site and the non-cutting area proximal to the surgical site. In this embodiment, the scalpel handle further contains a flat integrated lens in the surface proximate the bayonet with universal blade holder to direct light directly into the surgical site without obstruction from any blade attachment to the bayonet with universal blade holder.

In one embodiment, an illumination source is disposed at the base of the bayonet with universal blade holder to create a light transmissible scalpel handle with integrated lenses in both the body of the handle and the bayonet with universal blade holder. In this embodiment, light from one or more illumination sources is transmitted, amplified, diverged and/or converged through the bayonet with universal blade holder according to the shape of the lenses that are integrated into the scalpel handle and the portion of the bayonet with universal blade holder proximate the scalpel handle. A flat integrated lens is predisposed within or proximate to the tip of the bayonet with universal blade holder.

In one embodiment of the invention, the scalpel handle with one or multiple light sources in the handle body and the bayonet is substantially entirely light transmissible so that illumination sources and indicator lights can be viewed from the exterior of the scalpel handle and can transmit light from any illumination sources and indicator lights through the scalpel handle.

The scalpel handle and bayonet with universal blade holder may comprise an instrument, device, or medical device. The universal blade fitting attached to the bayonet can accommodate a variety of scalpel blades or other surgical instruments as is known in the art. The scalpel handle and bayonet with universal blade holder may be used on or in the body for treating, excising, incising or diagnosing including but not limited to skin hooks, dental mirrors, retractors, elevators, probes, curettes, or other instruments.

The scalpel handle, bayonet with universal blade holder and integrated lenses may comprise a wide variety of materials of construction. In one embodiment, the scalpel handle, the bayonet with universal blade holder and one or more of the integrated lenses comprises a light transmissible material. The light transmissible material may comprise optical glass, crystals, plastics, mirrors and combinations thereof.

In one embodiment, the scalpel handle is made from a transparent material at the end proximate the bayonet with universal blade holder and from an opaque or translucent material at the end distal the bayonet with universal blade holder. The bayonet with universal blade holder is attached to an end of the scalpel handle by any method now known or later developed. In one embodiment, the bayonet with universal blade holder may be permanently attached to the end of the scalpel handle. For example, the bayonet with universal blade holder may be molded or integrated as a single piece with the scalpel handle. In one embodiment, the bayonet with universal blade holder may be removably attached to the scalpel handle.

In one embodiment, the scalpel handle and bayonet with universal blade holder are disposable. In one embodiment, the scalpel handle and bayonet with universal blade holder may comprise a reusable material of construction that can be sterilized following surgery for reuse.

In one embodiment, a transparent scalpel handle and bayonet with universal blade holder comprises a circuit including one or more batteries, wires, PCB mounted resistors, one or more PCBs and one or more illumination sources mounted to the one or more PCBs disposed in the interior of the scalpel handle. In one embodiment, the one or more illumination sources may be used to pass light through the scalpel handle and/or bayonet holder with universal blade holder in a multitude of directions. An on/off switch is provided to complete a circuit between the batteries and the PCB and power the one or more illumination sources. Additional light sources may be present such as indicator lights that indicate the status of illumination operations, such as on and off positions.

In one embodiment, batteries, resistors and wires are contained within the hollow interior of the scalpel handle where the wires connect to the leads of one or more LED light sources. An on/off switch is provided to complete a circuit between the batteries and power the one or more illumination sources.

In one embodiment, an illumination source is disposed between two convex lenses integrated into the body of the scalpel handle that create a well to fit and secure the light source. In one embodiment, one or more illumination sources disposed in the scalpel handle at the end proximate the bayonet with universal blade holder is positioned within the well created by the shape of a circumferential convex lens, thus holding the one or more illumination sources in place to prevent it from moving when the scalpel handle and bayonet with universal blade holder are in use to perform surgery.

In one embodiment, one or more batteries provide power to illuminate the one or more illumination sources. In one embodiment, the one or more batteries are mounted on a PCB.

In one embodiment of the invention, the scalpel handle with one or multiple light sources in the handle body and the bayonet is entirely light transmissible so that PCB mounted illumination sources and indicator lights or non-PCB mounted illumination sources can be viewed from the exterior of the scalpel handle and can transmit light sources and indicator lights through the handle.

In one embodiment, the on/off switch comprises a device that interrupts an electrical connection between the batteries. In one embodiment, the device to interrupt the connection between the batteries comprises a tape disposed between the batteries and electrical connections that complete a circuit. In one embodiment, one end of the tape extends outward from the scalpel handle through a slot. To turn on the one or more illumination sources, the tape is removed thus completing the electrical connection between the batteries.

In one embodiment, a strip of a light transmissible material may be inserted between the interior walls of the scalpel handle and the top and bottom of the PCB to maintain a good electrical connection between the batteries and the PCB circuit. In one embodiment, the PCB is a flexible PCB. In this embodiment, one or more batteries are in contact with the PCB in a series formation.

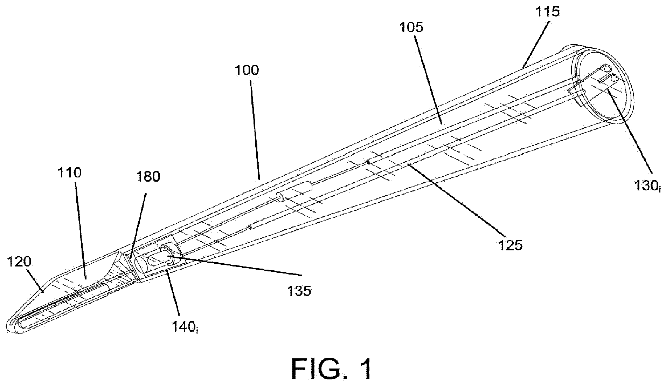

Turning to the drawings, FIG. 1 is a perspective view of a scalpel handle 100 and bayonet with universal blade holder 110 and light pipe 120 according to one embodiment of the invention. Scalpel handle 100 comprises a hollow interior cavity 105 and outer wall 115. Circuit 125 is provided in hollow interior cavity 105. Circuit 125 is connected to a plurality of batteries 130i. One or more integrated lenses 140i are integrated into the outer walls of scalpel handle 100. Illumination source 135 is disposed in hollow interior cavity 105 adjacent bayonet with universal blade holder 110. A substantially flat lens 180 is disposed in hollow interior cavity 105 between illumination source 135 and bayonet with universal blade holder 110.

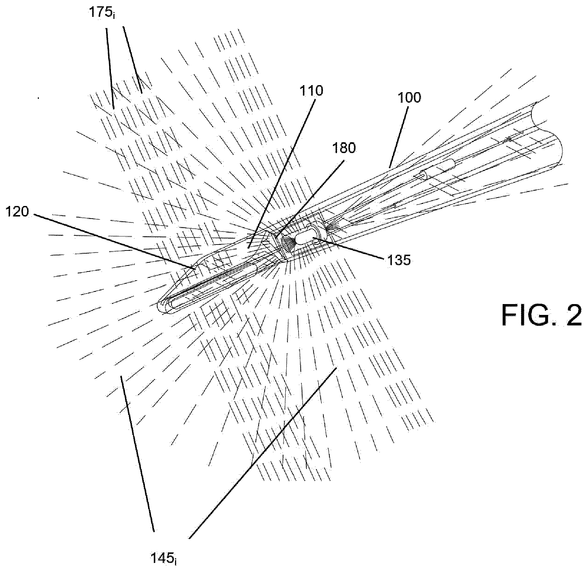

Fig 2 is a perspective view of scalpel handle 100 and bayonet with universal blade holder 110 of FIG. 1. When circuit 125 is completed to provide power to batteries 130i, illumination source 135 emits light 145i which emanates through transparent scalpel handle 100 including one or more integrated lenses 140i and light 175i which emanates through light pipe 120.

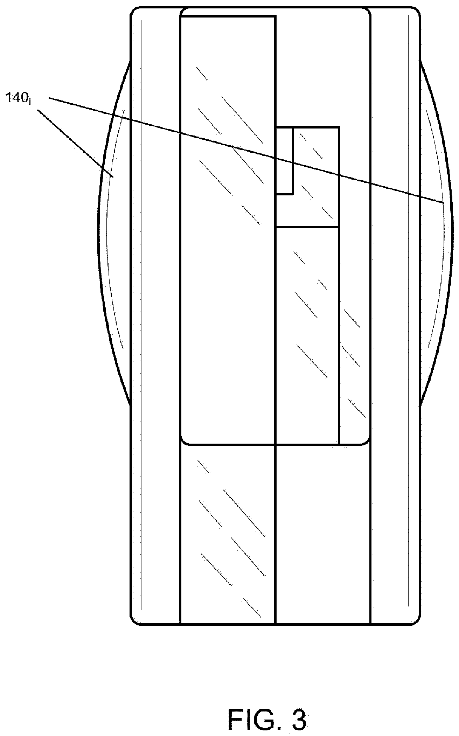

FIG. 3 depicts a front view of integrated lenses 140i, in this embodiment comprising convex lenses.

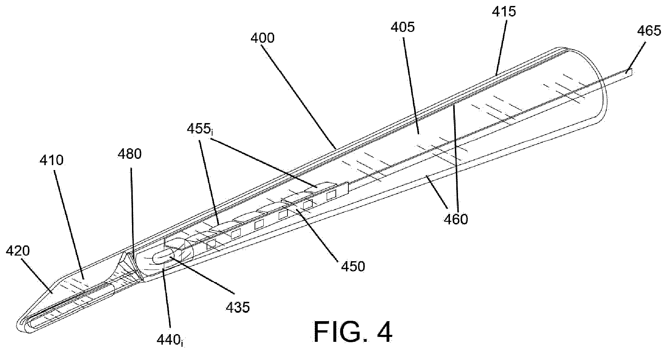

FIG. 4 is a perspective view of a scalpel handle 400 and bayonet with universal blade holder 410 and light pipe 420 according to one embodiment of the invention. Scalpel handle 400 comprises a hollow interior cavity 405 and outer wall 415. Flex PCB 450 is provided in hollow interior cavity 405. A plurality of batteries 455i are in contact with flex PCB 450. One or more integrated lenses 440i are integrated into the outer walls of scalpel handle 400. A strip of light transmissible material 460 is disposed between outer wall of transparent scalpel handle 400 and flex PCB 450 to maintain good electrical contact between flex PCB 450 and plurality of batteries 555i. An on/off switch 465 comprises a strip of tape that initially is disposed between at least one side of batteries 455i and flex PCB 450, preventing completion of an electrical circuit. A substantially flat lens 480 is disposed in hollow interior cavity 405 between illumination source 435 and bayonet with universal blade holder 410.

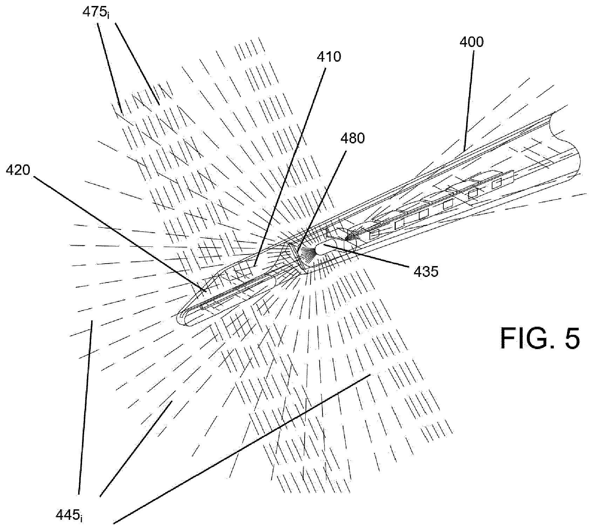

Fig 5 is a perspective view of scalpel handle 400 and bayonet with universal blade holder 410 of FIG. 4. When tape 465 is removed, a circuit is completed to provide power to batteries 455i, illumination source 435 emits light 445i which emanates through transparent scalpel handle 400 including one or more integrated lenses 440i and light 475i which emanates through light pipe 420.

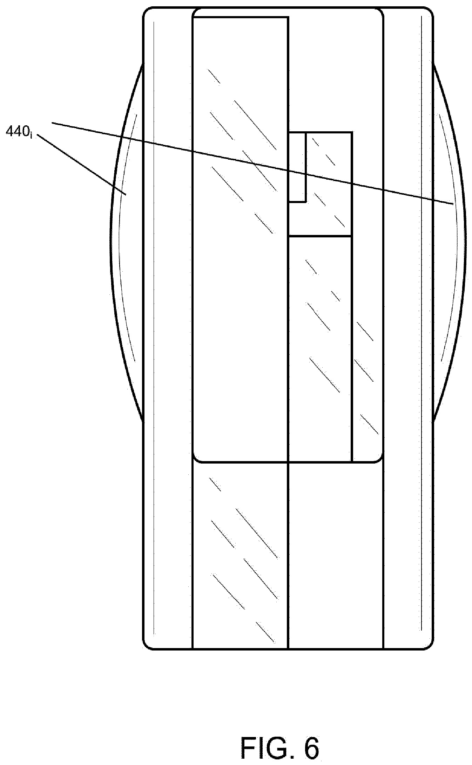

FIG. 6 depicts a front view of integrated lenses 440i in this embodiment comprising convex lenses.

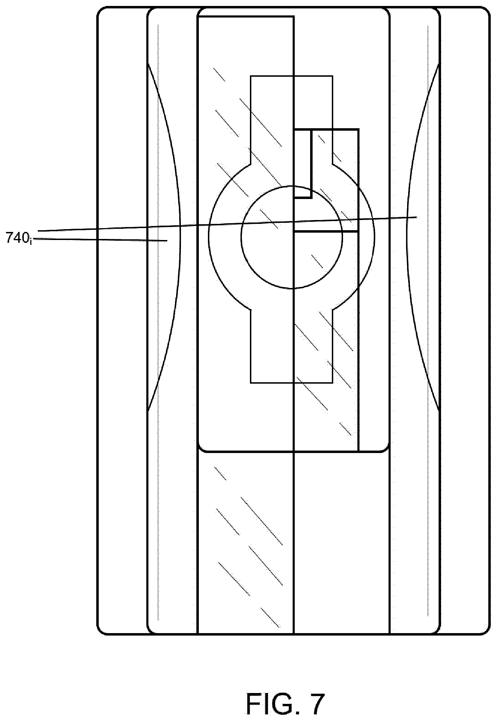

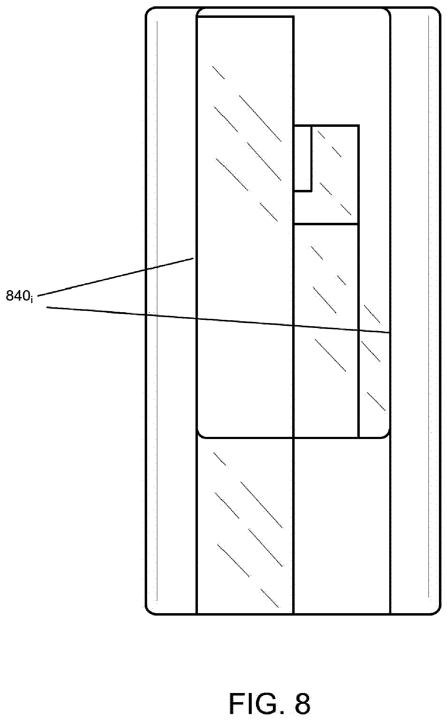

FIGS. 7 and 8 depict a front view of alternative profiles of integrated lenses suitable for use comprising concave and flat integrated lenses 740i and 840i, respectively.

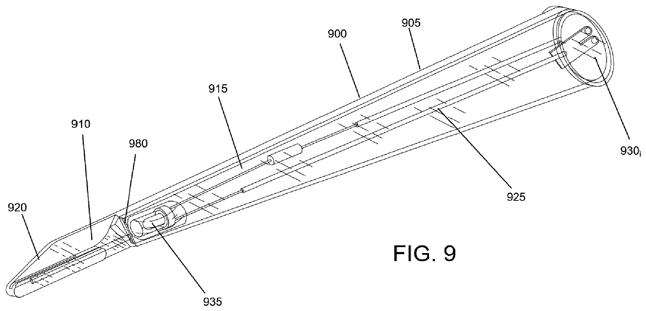

FIG. 9 is a perspective view of a scalpel handle 900 and bayonet with universal blade holder 910 and light pipe 920**.** Scalpel handle 900 comprises a hollow interior cavity 905 and outer wall 915. Circuit 925 is provided in hollow interior cavity 905. Circuit 925 is connected to a plurality of batteries 930i. A substantially flat lens 980 is disposed in hollow interior cavity 905 between illumination source 935 and bayonet with universal blade holder 910.

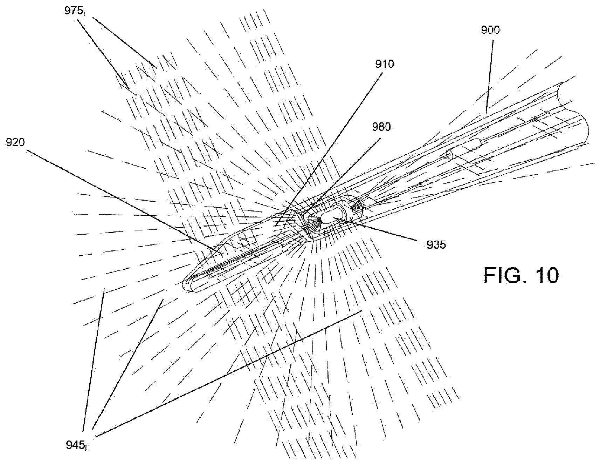

Fig 10 is a perspective view of scalpel handle 900 and bayonet with universal blade holder 910 of FIG. 9. When circuit 925 is completed to provide power to batteries 930i, illumination source 935 emits light 945i which emanates through transparent scalpel handle 900 and light 975i which emanates through light pipe 920.

## Claims

1. A scalpel handle and bayonet with universal blade holder, comprising:
- a scalpel handle (100, 400, 900), the scalpel handle comprising an outer wall (115, 415), the outer wall defining an interior hollow cavity (105, 405);
- a bayonet (110, 410) attached to an end of the scalpel handle in longitudinal coordination, the bayonet comprising a universal blade fitting;
- an electrical circuit (125) disposed in the interior hollow cavity of the scalpel handle;
- a power source (130i, 455i) disposed in the interior hollow cavity in communication with the electrical circuit;
- one or more illumination sources (135, 435) disposed in the interior hollow cavity in communication with the electrical circuit; and
- a switch that completes the electrical circuit;
wherein the scalpel handle and the bayonet comprise at least in part a transparent material;
wherein upon activation of the switch, the electrical circuit is completed between the power source and the one or more illumination sources causing the one or more illumination sources to emit light (145i, 175i, 445i, 475i);
wherein the scalpel handle and bayonet comprise one or more lenses (140i, 180, 440i, 480) integrated in at least one of the scalpel handle, the bayonet, the universal blade holder and combinations thereof;
wherein the scalpel handle is made from a transparent material at least at the end proximate the bayonet with universal blade holder;
wherein two convex lenses (140i, 440i) are disposed in the outer wall of the scalpel handle adjacent one of the one or more illumination sources, forming a well to secure the one or more illumination sources from movement, **and** wherein a substantially flat lens (180, 480) is disposed in the interior hollow cavity between the illumination source and the bayonet with universal blade holder.

2. The scalpel handle and bayonet with universal blade holder of claim 1, wherein the power source comprises one or more batteries (130i, 455i).

3. The scalpel handle and bayonet with universal blade holder of claim 1, wherein the one or more illumination sources (135, 435) comprises one or more light bulbs.

4. The scalpel handle and bayonet with universal blade holder of claim 3, wherein the one or more light bulbs comprises one or more LED bulbs.

5. The scalpel handle and bayonet with universal blade holder of claim 4, wherein the LED bulbs emit blue light having a wavelength of between about 380 nm and 500 nm.

6. The scalpel handle and bayonet with universal blade holder of claim 1, wherein the electrical circuit comprises a PCB.

7. The scalpel handle and bayonet with universal blade holder of claim 6, wherein the one or more illumination sources are attached to the PCB.

8. The scalpel handle and bayonet with universal blade holder of claim 6, wherein the PCB comprises a flexible PCB (450).

9. The scalpel handle and bayonet with universal blade holder of claim 1, wherein the switch comprises an on/off switch.

10. The scalpel handle and bayonet with universal blade holder of claim 1, wherein the switch comprises a material that interrupts a connection between the one or more power sources and the electrical circuit.

11. The scalpel handle and bayonet with universal blade holder of claim 10, wherein the material that interrupts the connection between the one or more power sources and the electrical circuit comprises a tape (465), wherein the tape extends through a slot in the hollow interior cavity to the exterior of the scalpel handle when the connection between the one or more power sources and the electrical circuit is interrupted.

12. The scalpel handle and bayonet with universal blade holder of claim 11, wherein the tape (465) is removed through the slot to complete the connection between the one or more power sources and the electrical circuit.

13. The scalpel handle and bayonet with universal blade holder of claim 1, wherein the scalpel handle comprises a reusable sterilizable material.

14. The scalpel handle and bayonet with universal blade holder of claim 1, wherein the bayonet and universal blade holder comprises a reusable sterilizable material.

15. The scalpel handle and bayonet with universal blade holder of claim 1, wherein the scalpel handle comprises a disposable sterilizable material.

16. The scalpel handle and bayonet with universal blade holder of claim 1, wherein the bayonet and universal blade holder comprises a disposable sterilizable material.

17. The scalpel handle and bayonet with universal blade holder of claim 1, further comprising one or more light pipes (120, 420) integral with the bayonet with universal blade fitting, wherein each of the one or more light pipes are in communication with one of the one or more illumination sources (135, 435) such that light emitted by the one or more illumination sources is transmitted by one or more of the light pipes.

18. The scalpel handle and bayonet with universal blade holder of claim 17, wherein one or more of the light pipes (120, 420) are in direct communication with one or more of the illumination sources (135, 435).

19. The scalpel handle and bayonet with universal blade holder of claim 118, wherein the bayonet with universal blade holder is removably attached to the end of the scalpel handle.

## Patentansprüche

1. Ein Skalpellgriff und ein Bajonett mit Universal-Klingenhalter, bestehend aus:
- einem Skalpellgriff (100, 400, 900), wobei der Skalpellgriff eine Außenwand (115, 415) umfasst, die einen inneren Hohlraum (105, 405) begrenzt;
- einem Bajonett (110, 410), das in Längsrichtung an einem Ende des Skalpellgriffs befestigt ist, wobei das Bajonett eine Universal-Klingenhalterung umfasst;
- einem elektrischen Schaltkreis (125), der im inneren Hohlraum des Skalpellgriffs angeordnet ist;
- einer Stromquelle (130i, 455i), die im inneren Hohlraum angeordnet ist und mit dem Schaltkreis verbunden ist;
- einer oder mehrerer Lichtquellen (135, 435), die im inneren Hohlraum angeordnet sind und mit dem elektrischen Schaltkreis verbunden sind; und
- einem Schalter, der den Stromkreis schließt;
wobei der Skalpellgriff und das Bajonett zumindest teilweise aus einem transparenten Material bestehen;
wobei bei Betätigung des Schalters der Schaltkreis zwischen der Stromquelle und der einen oder den mehreren Lichtquellen geschlossen wird, wodurch die eine oder mehrere Lichtquellen Licht ausstrahlen (145i, 175i, 445i, 475i);
wobei der Skalpellgriff und das Bajonett eine oder mehrere Linsen (140i, 180, 440i, 480) umfassen, die in mindestens einem der folgenden Elemente integriert sind: dem Skalpellgriff, dem Bajonett, dem Universal-Klingenhalter oder Kombinationen dieser;
wobei der Skalpellgriff zumindest an dem dem Bajonett mit Universal-Klingenhalter benachbarten Ende aus einem transparenten Material besteht;
wobei zwei konvexe Linsen (140i, 440i) in der Außenwand des Skalpellgriffs neben einer oder mehrerer Lichtquellen angeordnet sind und eine Vertiefung bilden, um eine oder mehrere Lichtquellen gegen eine Bewegung zu sichern, und wobei eine im Wesentlichen flache Linse (180, 480) in dem inneren Hohlraum zwischen der Lichtquelle und dem Bajonett mit Universal-Klingenhalter angeordnet ist.

2. Der Skalpellgriff und das Bajonett mit Universal-Klingenhalter nach Anspruch 1, wobei die Energiequelle eine oder mehrere Batterien (130i, 455i) umfasst.

3. Der Skalpellgriff und das Bajonett mit Universal-Klingenhalter nach Anspruch 1, wobei eine oder mehrere Lichtquellen (135, 435) eine oder mehrere Lampen umfassen.

4. Der Skalpellgriff und das Bajonett mit Universal-Klingenhalter nach Anspruch 3, wobei eine oder mehrere Lampen eine oder mehrere LED-Lampen umfassen.

5. Der Skalpellgriff und das Bajonett mit Universal-Klingenhalter nach Anspruch 4, wobei die LED-Lampen blaues Licht mit einer Wellenlänge zwischen etwa 380 nm und 500 nm ausstrahlen.

6. Der Skalpellgriff und das Bajonett mit Universal-Klingenhalter nach Anspruch 1, wobei der elektrische Schaltkreis eine Leiterplatte umfasst.

7. Der Skalpellgriff und das Bajonett mit Universal-Klingenhalter nach Anspruch 6, wobei eine oder mehrere Lichtquellen an der Leiterplatte angebracht sind.

8. Der Skalpellgriff und das Bajonett mit Universal-Klingenhalter nach Anspruch 6, wobei die Leiterplatte eine flexible Leiterplatte (450) umfasst.

9. Der Skalpellgriff und das Bajonett mit Universal-Klingenhalter nach Anspruch 1, wobei der Schalter einen Ein-/Aus-Schalter umfasst.

10. Der Skalpellgriff und das Bajonett mit Universal-Klingenhalter nach Anspruch 1, wobei der Schalter ein Material umfasst, das eine Verbindung zwischen der einen oder mehreren Stromquellen und dem Schaltkreis unterbricht.

11. Der Skalpellgriff und das Bajonett mit Universal-Klingenhalter nach Anspruch 10, wobei das Material, das die Verbindung zwischen der einen oder mehreren Stromquellen und dem Schaltkreis unterbricht, aus einem Band (465) gesteht, wobei sich das Band durch einen Schlitz im inneren Hohlraum bis zur Außenseite des Skalpellgriffs erstreckt, wenn die Verbindung zwischen der einen oder mehreren Stromquellen und dem Schaltkreis unterbrochen ist.

12. Der Skalpellgriff und das Bajonett mit Universal-Klingenhalter nach Anspruch 11, wobei das Band (465) durch den Schlitz entfernt wird, um die Verbindung zwischen der einen oder mehreren Stromquellen und dem Schaltkreis herzustellen.

13. Der Skalpellgriff und das Bajonett mit Universal-Klingenhalter nach Anspruch 1, wobei der Skalpellgriff aus einem wiederverwendbaren, sterilisierbaren Material besteht.

14. Der Skalpellgriff und das Bajonett mit Universal-Klingenhalter nach Anspruch 1, wobei das Bajonett und der Universal-Klingenhalter aus einem wiederverwendbaren, sterilisierbaren Material bestehen.

15. Der Skalpellgriff und das Bajonett mit Universal-Klingenhalter nach Anspruch 1, wobei der Skalpellgriff aus einem sterilisierbaren Einwegmaterial besteht.

16. Der Skalpellgriff und das Bajonett mit Universal-Klingenhalter nach Anspruch 1, wobei das Bajonett und der Universal-Klingenhalter aus einem sterilisierbaren Einwegmaterial bestehen.

17. Der Skalpellgriff und das Bajonett mit Universal-Klingenhalter nach Anspruch 1, ferner umfassend einen oder mehrere Lichtleiter (120, 420), die einstückig mit dem Bajonett mit Universal-Klingenhalter ausgebildet sind, wobei jeder der einen oder mehreren Lichtleiter mit einer oder mehreren Lichtquellen (135, 435) in Verbindung steht, so dass das von der einen oder den mehreren Lichtquellen emittierte Licht durch einen oder mehrere der Lichtleiter übertragen wird.

18. Der Skalpellgriff und das Bajonett mit Universal-Klingenhalter nach Anspruch 17, wobei einer oder mehrere der Lichtleiter (120, 420) in direkter Verbindung mit einer oder mehreren Lichtquellen (135, 435) stehen.

19. Der Skalpellgriff und das Bajonett mit Universal-Klingenhalter nach Anspruch 18, wobei das Bajonett mit Universal-Klingenhalter abnehmbar am Ende des Skalpellgriffs befestigt ist.

## Revendications

1. Manche de scalpel et baïonnette avec support de lame universel, comprenant :
- un manche de scalpel (100, 400, 900), ledit manche de scalpel comprenant une paroi extérieure (115, 415), ladite paroi extérieure délimitant une cavité intérieure creuse (105, 405) ;
- une baïonnette (110, 410) fixée à une extrémité du manche de scalpel dans le prolongement longitudinal de celui-ci, ladite baïonnette comprenant un support de lame universel ;
- un circuit électrique (125) disposé dans la cavité intérieure creuse du manche de scalpel ;
- une source d'alimentation (130i, 455i) disposée dans la cavité intérieure creuse et en communication électrique avec ledit circuit électrique ;
- une ou plusieurs sources lumineuses (135, 435) disposées dans la cavité intérieure creuse et en communication électrique avec ledit circuit électrique ; et
- un interrupteur apte à fermer le circuit électrique ;
dans lequel le manche de scalpel et la baïonnette sont constitués, au moins en partie, d'un matériau transparent ;
dans lequel, lors de l'actionnement de l'interrupteur, le circuit électrique est fermé entre la source d'alimentation et lesdites une ou plusieurs sources lumineuses, de manière à provoquer l'émission de lumière (145i, 175i, 445i, 475i) par lesdites une ou plusieurs sources lumineuses ;
dans lequel le manche de scalpel et la baïonnette comprennent une ou plusieurs lentilles optiques (140i, 180, 440i, 480) intégrées dans au moins l'un parmi le manche de scalpel, la baïonnette, le support de lame universel et leurs combinaisons ;
dans lequel le manche de scalpel est réalisé en matériau transparent au moins au niveau de son extrémité située à proximité de la baïonnette avec support de lame universel ;
et dans lequel deux lentilles convexes (140i, 440i) sont disposées dans la paroi extérieure du manche de scalpel à proximité de l'une desdites une ou plusieurs sources lumineuses, de manière à former un logement destiné à maintenir ladite source lumineuse ou lesdites sources lumineuses en position, **tandis** qu'une lentille sensiblement plane (180, 480) est disposée dans la cavité intérieure creuse entre la source lumineuse et la baïonnette avec support de lame universel.

2. Manche de scalpel et baïonnette avec support de lame universel selon la revendication 1, **caractérisés en ce que** la source d'alimentation comprend une ou plusieurs piles (130i, 455i).

3. Manche de scalpel et baïonnette avec support de lame universel selon la revendication 1, **caractérisés en ce que** lesdites une ou plusieurs sources lumineuses (135, 435) comprennent une ou plusieurs ampoules.

4. Manche de scalpel et baïonnette avec support de lame universel selon la revendication 3, **caractérisés en ce que** lesdites une ou plusieurs ampoules comprennent une ou plusieurs diodes électroluminescentes (DEL).

5. Manche de scalpel et baïonnette avec support de lame universel selon la revendication 4, **caractérisés en ce que** lesdites diodes électroluminescentes émettent une lumière bleue présentant une longueur d'onde comprise entre environ 380 nm et 500 nm.

6. Manche de scalpel et baïonnette avec support de lame universel selon la revendication 1, **caractérisés en ce que** le circuit électrique comprend une carte de circuit imprimé (PCB).

7. Manche de scalpel et baïonnette avec support de lame universel selon la revendication 6, **caractérisés en ce que** lesdites une ou plusieurs sources lumineuses sont fixées à la carte de circuit imprimé (PCB).

8. Manche de scalpel et baïonnette avec support de lame universel selon la revendication 6, **caractérisés en ce que** la carte de circuit imprimé comprend une carte de circuit imprimé flexible (450).

9. Manche de scalpel et baïonnette avec support de lame universel selon la revendication 1, **caractérisés en ce que** l'interrupteur comprend un interrupteur marche/arrêt.

10. Manche de scalpel et baïonnette avec support de lame universel selon la revendication 1, **caractérisés en ce que** l'interrupteur comprend un matériau interrompant une connexion entre la ou les sources d'alimentation et le circuit électrique.

11. Manche de scalpel et baïonnette avec support de lame universel selon la revendication 10, **caractérisés en ce que** le matériau interrompant la connexion entre la ou les sources d'alimentation et le circuit électrique comprend une bande (465), ladite bande s'étendant, lorsque la connexion entre la ou les sources d'alimentation et le circuit électrique est interrompue, à travers une fente ménagée dans la cavité intérieure creuse jusqu'à l'extérieur du manche de scalpel.

12. Manche de scalpel et baïonnette avec support de lame universel selon la revendication 11, **caractérisés en ce que** la bande (465) est retirée à travers ladite fente afin d'établir la connexion entre la ou les sources d'alimentation et le circuit électrique.

13. Manche de scalpel et baïonnette avec support de lame universel selon la revendication 1, **caractérisés en ce que** le manche de scalpel comprend un matériau stérilisable réutilisable.

14. Manche de scalpel et baïonnette avec support de lame universel selon la revendication 1, **caractérisés en ce que** la baïonnette et le support de lame universel comprennent un matériau stérilisable réutilisable.

15. Manche de scalpel et baïonnette avec support de lame universel selon la revendication 1, **caractérisés en ce que** le manche de scalpel comprend un matériau stérilisable jetable.

16. Manche de scalpel et baïonnette avec support de lame universel selon la revendication 1, **caractérisés en ce que** la baïonnette et le support de lame universel comprennent un matériau stérilisable jetable.

17. Manche de scalpel et baïonnette avec support de lame universel selon la revendication 1, comprenant en outre un ou plusieurs guides de lumière (120, 420) solidaires de la baïonnette avec support de lame universel, chacun desdits guides de lumière étant en communication optique avec l'une desdites une ou plusieurs sources lumineuses (135, 435), de telle sorte que la lumière émise par lesdites une ou plusieurs sources lumineuses soit transmise par un ou plusieurs desdits guides de lumière.

18. Manche de scalpel et baïonnette avec support de lame universel selon la revendication 17, **caractérisés en ce qu'**un ou plusieurs desdits guides de lumière (120, 420) sont en communication directe avec une ou plusieurs desdites sources lumineuses (135, 435).

19. Manche de scalpel et baïonnette avec support de lame universel selon la revendication 18, **caractérisés en ce que** la baïonnette avec support de lame universel est fixée de manière amovible à l'extrémité du manche de scalpel.
